(19) Europäisches Patentamt
European Patent Office
Office européen des brevets

(11) EP 4 201 940 A1

(12) # EUROPEAN PATENT APPLICATION

(43) Date of publication:
**28.06.2023 Bulletin 2023/26**

(21) Application number: **21306902.4**

(22) Date of filing: **22.12.2021**

(51) International Patent Classification (IPC):
***C07F 1/00*** *(2006.01)* ***C07F 3/00*** *(2006.01)*
***C07C 63/331*** *(2006.01)* ***C07C 65/105*** *(2006.01)*
***C07C 65/24*** *(2006.01)* ***B82Y 40/00*** *(2011.01)*

(52) Cooperative Patent Classification (CPC):
(C-Sets available)
**C07F 1/005; C07C 41/16; C07C 51/09; C07C 67/343; C07F 3/003;** B82Y 40/00 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Université de Strasbourg**
  **67000 Strasbourg (FR)**
- **Centre national de la recherche scientifique**
  **75016 Paris (FR)**

(72) Inventors:
- **LOUIS, Benoît**
  **67118 Geispolsheim (FR)**
- **ISRAFILOV, Nizami**
  **67100 Strasbourg (FR)**
- **PLANEIX, Jean-Marc**
  **67000 Strasbourg (FR)**

(74) Representative: **Novagraaf Technologies**
**Bâtiment O2**
**2, rue Sarah Bernhardt**
**CS90017**
**92665 Asnières-sur-Seine Cedex (FR)**

(54) **SYNTHESIS OF METAL ORGANIC FRAMEWORK (MOF) MATERIALS WITH HIGH ADSORPTION CAPACITY OF ORGANIC COMPOUNDS AND CO2 CAPTURE**

(57) Metal-organic framework material (MOF), comprising at least one metal center and at least one organic compound selected among the compounds according to formula (I):

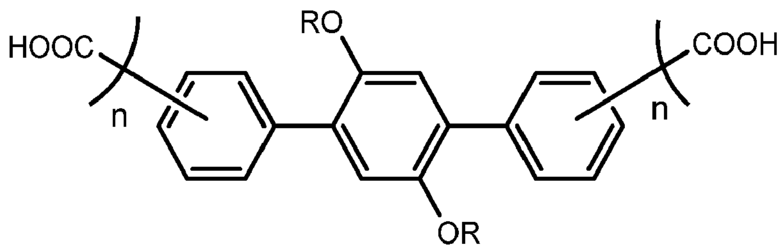

(I)

- wherein n is from 1 to 2,
- R is $-(CH_2-CH_2-O)_m-CH_3$ with m from 1 to 7;

preferably when n=1, the two -COOH groups are located in the para position relatively to the aryl substituted by the two RO groups; and
preferably when n=2, the four -COOH groups are located in the meta position relatively to the aryl substituted by the two RO groups.

EP 4 201 940 A1

FIG. 1

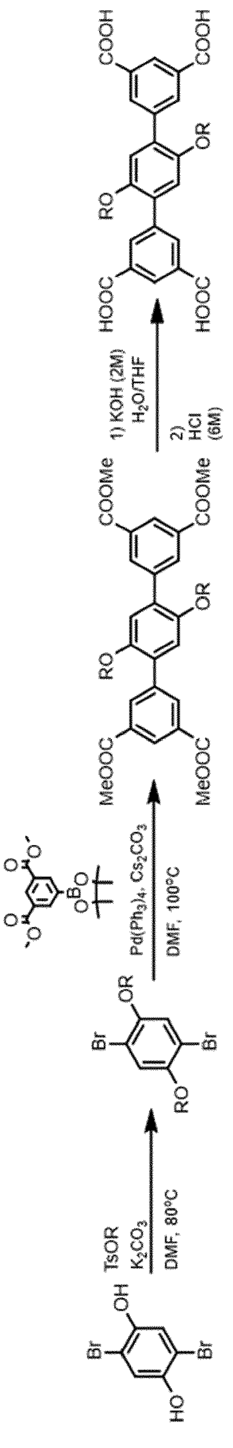


1: R=$(CH_2CH_2O)CH_3$

2: R= $(CH_2CH_2O)_2CH_3$

3: R=$(CH_2CH_2O)CH_3$

4: R= $(CH_2CH_2O)_2CH_3$

5: R=$(CH_2CH_2O)CH_3$

6: R= $(CH_2CH_2O)_2CH_3$

(52) Cooperative Patent Classification (CPC): (Cont.)

C-Sets
**C07C 41/16, C07C 43/225;**
**C07C 51/09, C07C 65/24;**
**C07C 67/343, C07C 69/94**

## Description

### FIELD OF THE INVENTION

The present invention relates to aryl compounds comprising carboxylate groups and functionalized with ether groups, and to their association with metals in order to obtain crystalline solid materials, and more particularly to obtain metal organic frameworks or MOFs.

In what follows, the numbers between brackets (**[ ]**) refer to the List of References provided at the end of the document.

### BACKGROUND OF THE INVENTION

The use of amine-based liquids for carbon dioxide ($CO_2$) capture and storage is well known. Nevertheless, these liquids are toxic, corrosive and require a lot of energy to release the adsorbed $CO_2$ for regeneration, the search for an alternative has attracted much interest.

The use of metal organic frameworks (MOFs) compounds has been widely investigated for the capture of small molecules such as $CO_2$. Metal-organic frameworks (MOFs), also known as porous coordination polymers (PCPs) or coordination networks, are crystalline materials that can be easily self-assembled from metal ions or metal clusters with organic ligands. These MOFs are already extensively used in industry for the preparation of a large scale of materials, and they are particularly convenient for the preparation of new materials as MOFs can be processed and functionalized to impart new physical and chemical properties, charge conduction, and adjustable porosity.

MOF materials are solid adsorbents that are neither corrosive nor toxic and can be easily regenerated by known pressure swing adsorption techniques, or temperature swing adsorption techniques. Besides MOFs are porous coordination polymers, having an inorganic-organic hybrid framework that comprises metal ions and organic ligands coordinated to the metal ions. These materials are organized into one-, two- or three-dimensional lattices, in which the metallic species are joined together periodically by spacer ligands. The framework of these solids has both inorganic parts and organic parts, the cavities of which may be occupied by water molecules or by organic molecules that are easy to extract without deterioration of the backbone.

The use of aryl ligands incorporated into the MOF as the main component of its organic part has been investigated for the absorption of small molecules such as $H_2$ or $CO_2$. Reference **[1]** describes terphenyl-tetracarboxylate ligands with alkoxy functions containing alkyl side chains for the synthesis of MOFs for $H_2$ adsorption. Besides, in **[2]** other terphenyl-tetracarboxylate ligands with alkoxy functions containing alkyl side chains for the synthesis of MOFs are reported for the adsorption of $CO_2$, $H_2$, $N_2$ and methane.

There remains a need for the development of new synthetic processes which allow the preparation of MOF exhibiting enhanced properties of $CO_2$ adsorption or other organic and/or inorganic molecules such as dye, and with properties that outperform those of all the reported MOFs so far in this respect.

### BRIEF DESCRIPTION OF THE INVENTION

The present invention relates to an organic compound for the preparation of a MOF, said organic compound being selected among the compounds according to formula (I):

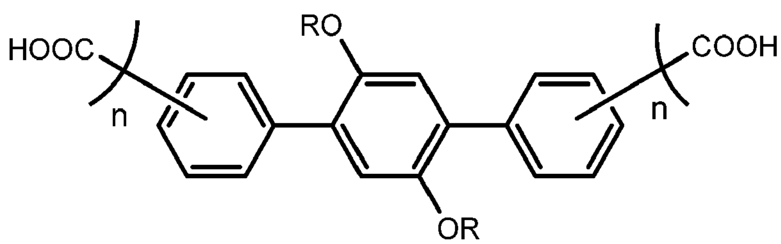

(I)

- wherein n is from 1 to 2,
- R is $-(CH_2-CH_2-O)_m-CH_3$ with m from 1 to 7;

preferably when n=1, the two -COOH groups are located in the para position relatively to the aryl substituted by the two RO groups; and

preferably when n=2, the four -COOH groups are located in the meta position relatively to the aryl substituted by the two RO groups.

The inventors have demonstrated unexpectidly that MOF comprising ligands consisting of those organic compounds provides hight stability in water, with great thermostability as they are stable up to 300°C and even under water steam, and that $CO_2$ absorption or dye molecules such as methylene blue was better compared to the MOFs described in the previous art.

Advantageously, n is 2, the organic compound being selected among the compounds according to formula (II):

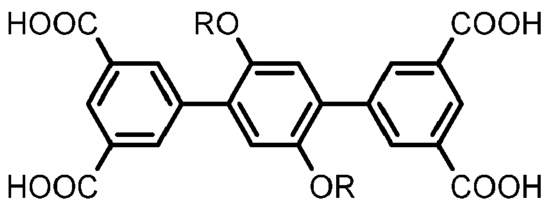

(II)

and preferably, m is less than 4.

The present invention relates also to a metal-organic framework material (MOF), comprising at least one metal center and at least one organic compound as previously described in the context of the present invention.

Advantageously, the metal center of the hydrophobic core is selected from Li, Na, Rb, Mg, Ca, Sr, Ba, Sc, Ti, Zr, Ta, Cr, Mo, W, Mn, Fe, Ru, Os, Co, Ni, Pd, Pt, Cu, Au, Zn, Al, Ga, In, Si, Ge, Sn, Bi, Cd, Mn, Tb, Gd, Ce, La, or Cr, and preferably Cu, Zn, Zr, Ca or Mg.

Advantageously, the organic compound of the MOF is :

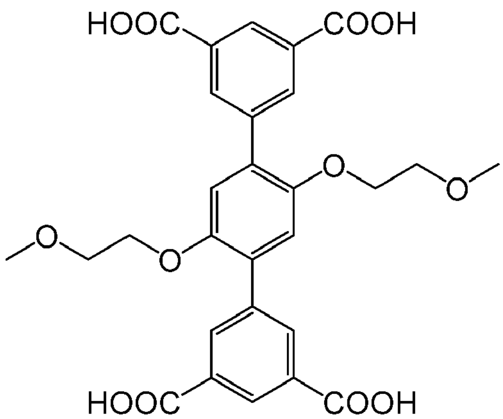

and the metal center is selected from Cu, Zn, Mg, Ni and Ca.

Advantageously, the organic compound of the MOF is selected among :

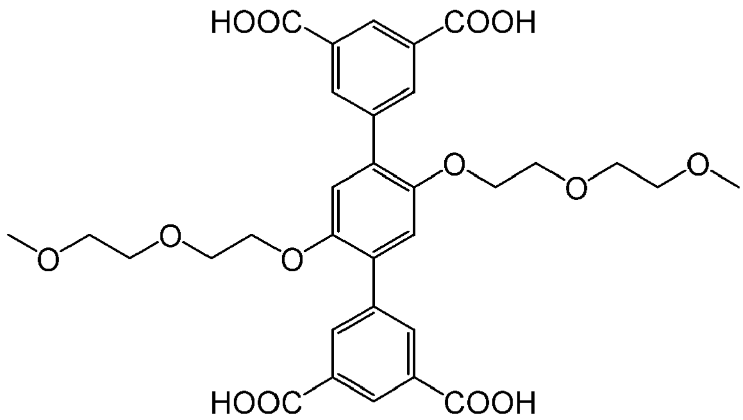

;

and

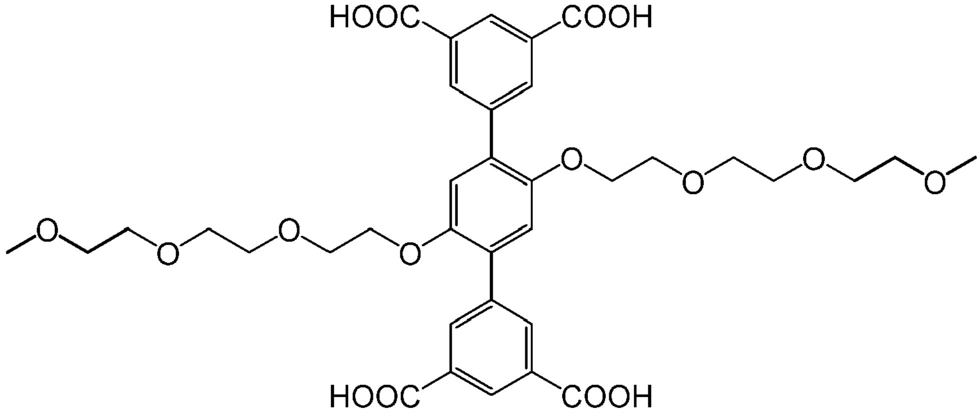

and the metal center is selected from Cu, Zn, Mg, Ni and Ca.

Advantageously, the organic compound is :

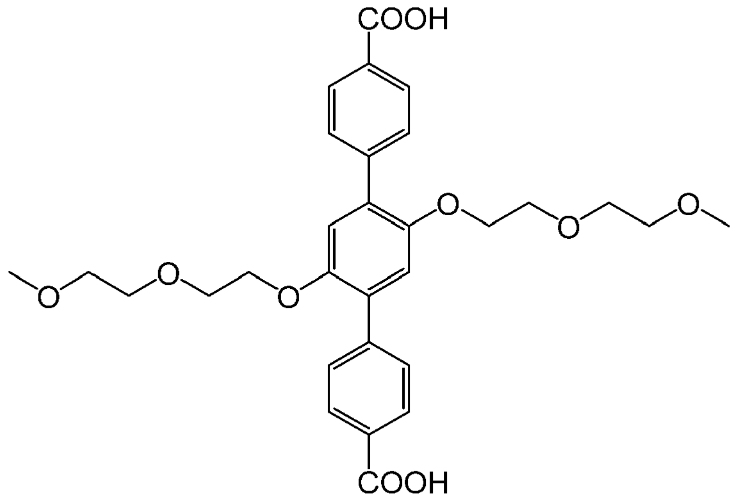

and the metal center is selected from Cu, Zr, Zn, Fe and Ca.

The present invention relates also to the use of a MOF as previously described in the context of the present invention, for the adsorption of volatile organic compounds (VOCs), and preferably for the adsorption of acetic acid or aldehydes.

Besides, the present invention relates to the use of a MOF as previously described in the context of the present invention, for the decontamination of aqueous solutions containing organic pollutants such as dyes and pharmaceutical

compounds, and preferably for the adsorption of a dye, preferably selected among a cationic and an anionic dye, and most preferably selected among at least methylene blue and alizarin yellow R.

In addition, the present invention relates to the use of a MOF as previously described in the context of the present invention, for the adsorption of a gas, preferably selected among methane, hydrogen, acetylene, nitrogen, and $CO_2$, most preferably $CO_2$. Said MOF can advantageously be used for:

- the separation of gases, for example for treating a gaz mixture containing methane and $CO_2$;
- realizing a gas phase catalysis, such as the preparation of methanol starting to CO. The concentration of adsorbed gas by the MOF used in the context of the invention for the adsorption of $CO_2$ is preferably greater than 17 mmol/g at 10 bars, and 0°C, and most preferably greater than 35 mmol/g at 10 bars, and 0°C.

The present invention relates also to a preparation method for the synthesis of a MOF as previously described in the context of the present invention, wherein the metal center is Cu and the MOF is prepared by the dissolution of the organic compound previously described in the context of the present invention, in a DMF/water mixture, followed by an addition plus dissolution of $Cu(NO_3)_2.3H_2O$ into the reaction mixture, the reaction mixture is left at 70-90°C during several hours in acidic conditions.

Advantageously, the synthesis of the organic compound comprises the steps of:

a- activating phenol groups of a hydroquinone bearing two halogen or two -OTf groups ;
b- performing a Suzuki-Miyaura reaction to replace halogen and/or -OTf by aryl groups bearing protected acid acetic substituents ; and
c- deprotecting the acid acetic substituents;

preferably according to the following synthetic pathway:

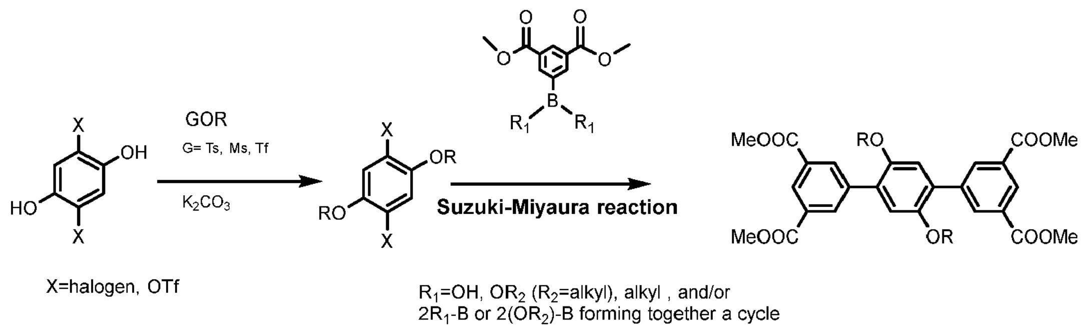


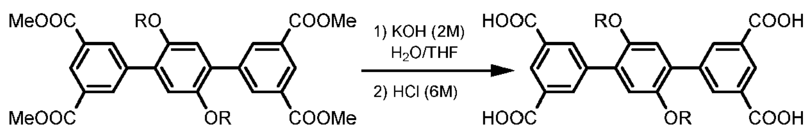


**(II)** ;

preferably, the X=Br; G=Ts and the first step of the synthetic pathway is realized in DMF at 80°C, and the Suzuki-Miyaura coupling reaction step is performed according to:

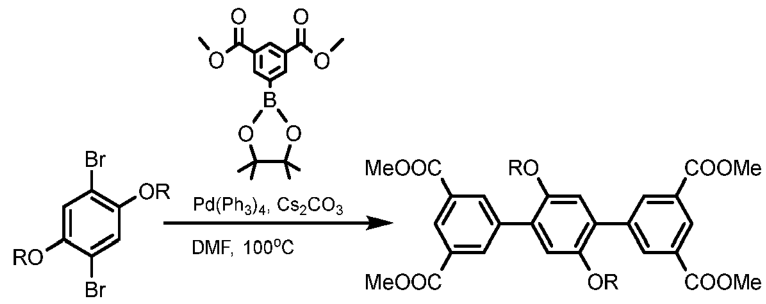


**(II)**

.

**DEFINITIONS**

To facilitate an understanding of the present invention, a number of terms and phrases are defined below:
The term "aliphatic", as used herein, includes both saturated and unsaturated, straight chain (i.e., unbranched) or branched aliphatic hydrocarbons, which are optionally substituted with one or more functional groups. As will be appreciated by one of ordinary skill in the art, "aliphatic" is intended herein to include, but is not limited to, alkyl, alkenyl, alkynyl moieties.

As used herein, the term "alkyl", refers to straight and branched alkyl groups. An analogous convention applies to other generic terms such as "alkenyl", "alkynyl" and the like. As used herein, "lower alkyl" is used to indicate those alkyl groups (substituted, unsubstituted, branched or unbranched) having about 1-6 carbon atoms. Illustrative alkyl groups include, but are not limited to, for example, methyl, ethyl, n-propyl, isopropyl, allyl, n-butyl, sec-butyl, isobutyl, tert-butyl, n-pentyl, sec-pentyl, isopentyl, tert-pentyl, n-hexyl, sec-hexyl, moieties and the like, which again, may bear one or more substituents. Alkenyl groups include, but are not limited to, for example, ethenyl, propenyl, butenyl, 1-methyl-2-buten-I-yl, and the like. Representative alkynyl groups include, but are not limited to, ethynyl, 2-propynyl (propargyl), 1-propynyl and the like.

In general, the term "aromatic" or "aryl", as used herein, refers to stable substituted or unsubstituted unsaturated mono- or polycyclic hydrocarbon moieties having preferably 3-14 carbon atoms, comprising at least one ring satisfying Hückle's rule for aromaticity. Examples of aromatic moieties include, but are not limited to, phenyl, indanyl, indenyl, naphthyl, phenanthryl and anthracyl.

As used herein, the term "heteroaryl" refers to unsaturated mono-heterocyclic or polyheterocyclic moieties having preferably 3-14 carbon atoms and at least one ring atom selected from S, O and N, comprising at least one ring satisfying the Hückel rule for aromaticity. The term "heteroaryl" refers to a cyclic unsaturated radical having from about five to about ten ring atoms of which one ring atom is selected from S, O and N; zero, one or two ring atoms are additional heteroatoms independently selected from S, O and N; and the remaining ring atoms are carbon, the radical being joined to the rest of the molecule via any of the ring atoms, such as, for example, pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, and the like. Examples of heteroaryl moieties include, but are not limited to, pyridyl, quinolinyl, dihydroquinolinyl, isoquinolinyl, quinazolinyl, dihydroquinazolyl, and tetrahydroquinazolyl.

As used herein, the expression "$C_x$-$C_y$, preferably $C_{x1}$-$C_{y1}$, alkylaryl, aralkyl or aryl", where x, y, x1 and y1 represent integers denoting the number of carbon atoms in the chemical moiety to which it refers (e.g., "alkylaryl", "aralkyl", "aryl")), means "$C_x$-$C_y$alkylaryl, $C_x$-$C_y$aralkyl or $C_x$-$C_y$aryl, preferably $C_{x1}$-$C_{y1}$alkylaryl, $C_{x1}$-$C_{y1}$ aralkyl or $C_{x1}$-$C_{y1}$aryl". Likewise, the expression "$C_x$-$C_y$ alkylaryl, aralkyl or aryl", means "$C_x$-$C_y$alkylaryl, $C_x$-$C_y$aralkyl or $C_x$-$C_y$aryl".

As used herein, the term "independently" refers to the fact that the substituents, atoms or moieties to which these terms refer, are selected from the list of variables independently from each other (i.e., they may be identical or the same).

One skilled in the art will also readily recognize that where members are grouped together in a common manner, such as in a Markush group, the invention encompasses not only the entire group listed as a whole, but each member of the group individually and all possible subgroups of the main group. Additionally, for all purposes, the invention encompasses not only the main group, but also the main group absent one or more of the group members. The invention therefore envisages the explicit exclusion of any one or more of members of a recited group. Accordingly, provisos may apply to any of the disclosed categories or embodiments whereby any one or more of the recited elements, species, or embodiments, may be excluded from such categories or embodiments, for example, as used in an explicit negative limitation.

## DETAILED DESCRIPTION OF CERTAIN PREFERRED EMBODIMENTS OF THE INVENTION Analysis and experimental protocols

**$^1$H-NMR and $^{13}$C-NMR** spectra were recorded at 25 °C on Brucker® AV300 (300 MHz) Bruker AV400 (400 MHz) or Bruker® AV500 (500 MHz) spectrometers in deuterated solvents with the residual solvent peak used as the internal reference ($CDCl_3$: 7.26 ppm for 1H, 77.2 ppm for $^{13}$C; DMSO-d6: 2.50 ppm for 1H, 49.9 ppm for $^{13}$C). The abbreviations for specifying the multiplicity of $^1$H-NMR signals are defined as follows: s = singlet, d= doublet, dd = doublet of doublet, ddd = doublet of doublet of doublets, t = triplet, q = quadruplet, m= multiplet, br = broad. Coupling constants are given in Hertz and chemical shifts in ppm.

**MS (Mass spectrometry)** was performed at the "Service de Spectrométrie de Masse" of the University of Strasbourg. Low (LRMS) (positive and negative mode ESI: Electro Spray Ionization) were recorded on Thermoquest AQA Navigator® with time flight detector.

**Elemental analyses** were performed on a Thermo Scientific Flash 2000 by the "Service Commun de Microanalyse" of the University of Strasbourg.

**UV-Vis spectrometry** measurements were performed on a Perkin-Elmer Lambda 900 spectrophotometer in 1mm quartz cuvettes. Wavelength ($\lambda$) are given in nm. All solvents used were purchased as spectrometric grades.

**Thermo gravimetric analysis (TGA)** were performed on a Pyris 6 TGA Lab System apparatus (Perkin-Elmer®), using a $N_2$ flow of 20 mL/min and a heat rate of 5°/min.

**X-Ray diffraction: Single-crystal** data were collected on a Bruker® SMART CCD diffractometer with Mo- K$\alpha$ radiation at 173 K. The structures were solved using SHELXS-97 and refined by full matrix least- squares on F2 using SHELXL-2014 with anisotropic thermal parameters for all non-hydrogen atoms.

The hydrogen atoms were introduced at calculated positions and not refined (riding model). The SQUEEZE command has been employed when disordered solvent molecules were present in structures, to account for the corresponding electron density.

**XRPD Diffraction patterns:** Powder X-ray diffraction (XRPD) data were recorded using a Bruker® D8 AV diffractometer with Cu-K$\alpha$ radiation at room temperature. The radiation wavelength $\lambda$ of the incident X-rays was 1,54 Å and a 2$\theta$ range is from 4° to 40° was investigated.

**BET measurements:** Nitrogen adsorption-desorption isotherms were measured at 77K up to 1 bar using the ASAP 2020 Micromeritics® analyzer. Carbon dioxide adsorption-desorption isotherms up to 10 bars at 273K were performed by using ASAP 2050 Micromeritics® analyzer.

The preparation of compounds **1** and **2** were realized according to protocols detailed in **[3].**

### Synthesis and characterization of organic compounds used as ligands in the MOF:

The preparation of the different organic compounds according to formula (II) was performed according to the scheme reported on figure 1.

- Preparation of compounds **1** or **2:** $TsO(CH_2\text{-}CH_2\text{-}O)\text{-}CH_3$ or $TsO(CH_2\text{-}CH_2\text{-}O)_2\text{-}CH_3$ (11.22 mmol), dibromohydroquinone (4.48 mmol), and potassium carbonate (26.88 mmol) were placed in a double necked bottom flask under inert atmosphere (Ar), and DMF solvent was added. The mixture was stirred overnight (18 h) at 80°C, then the reaction mixture was quenched with 120 ml water to obtain a white precipitate. Small amount of benzoquinone gives brownish color. The product was recrystallized to eliminate this color. Yield for compound **1**=53,6%. Compound **2**=71%.
- Preparation of compounds **3** or **4:** The dimethyl 5-(4,4,5,5-tetramethyl-1,3,2-dioxaborolan-2-yl)isophthalate (6.25 mmol) and compound **1** or **2** (2.08 mmol) were placed in a double necked bottom flask, then 40 ml DMF was added under Argon atmosphere. After 20 min mixing under argon, cesium carbonate (6.25 mmol) and tetrakis(triphenylphosphine)palladium (0) (0.054 mmol) were added, and the mixture was heated under stirring. Reaction continued overnight. Reaction mixture was then dried under reduced pressue, extracted with chloroform, and purified with column. A white solid was obtained.

Compound **3**: Yield=79%;
$^1$H NMR (500 MHz, $CDCl_3$) $\delta$ 8.67 (d, *J* = 1.7 Hz, 2H), 8.49 (d, *J* = 1.7 Hz, 4H), 7.07 (s, 2H), 4.13 (t, *J* = 4.7 Hz, 4H), 3.98 (s, 12H), 3.66 (t, *J* = 4.7 Hz, 4H), 3.34 (s, 6H);
$^{13}$C NMR (126 MHz, $CDCl_3$) $\delta$ 166.38, 150.39, 138.61, 134.90, 130.47, 129.71, 129.44, 116.28, 71.01, 69.32, 59.23, 52.47;
LRMS (ESI$^+$) calculated: $C_{32}H_{34}O_{12}$, 610.21 found: $C_{32}H_{34}O_{12}Na$, 633.19;
elemental analysis (%) for $C_{32}H_{34}O_{12}$ calculated: C 62.95, H 5.61; found: C 60.52, H 5.54.
Compound **4**: Yield=90%;

$^1H$ NMR (500 MHz, $CDCl_3$) δ 8.64 (t, J = 1.7 Hz, 2H), 8.44 (d, J = 1.7 Hz, 4H), 7.02 (s, 2H), 4.12 (t, J = 4.9 Hz, 4H), 3.96 (s, 13H), 3.74 (t, J = 4.9 Hz, 4H), 3.58-3.53 (m, 4H), 3.46 - 3.41 (m, 4H), 3.29 (s, 6H).
$^{13}C$ NMR (126 MHz, $CDCl_3$) δ 166.32, 150.28, 138.69, 134.88, 130.45, 129.65, 129.40, 116.06, 71.88, 70.79, 69.68, 69.42, 59.02, 52.47.
LRMS ($ESI^+$) calculated: $C_{36}H_{42}O_{14}$, 698.26, found $C_{36}H_{42}O_{14}Na$, 721.25;
elemental analysis (%) for calculated: C 61.88, H 6.06; found C 58.95, H 5.82. - Preparation of compounds **5** or **6:** compound **3** or **4** (1.64 mmol) was dissolved in the mixture of 100 ml THF and 100 ml aq. KOH (2M). The mixture was heated to 90°C overnight. The heating was stopped, and once reaction mixture reached RT, THF was evaporated under reduced pressure. The aqueous solution was treated with 6M HCl to pH1. The obtained precipitate was filtered and washed with water, plus dried under vacuum overnight.
Compound **5**: Yield=89%;
$^1H$ NMR (300 MHz, DMSO) δ : 13.29 (s, 4H), 8.46 (t, *J* = 1.6 Hz, 2H), 8.42 (d, *J* = 1.6 Hz, 4H), 7.25 (s, 2H), 4.24 - 4.15 (m, 4H), 3.63 - 3.54 (m, 4H), 3.22 (s, 6H);
$^{13}C$ NMR (126 MHz, DMSO) δ : 167.12, 150.23, 138.70, 134.67, 131.67, 129.22, 129.08, 116.25, 70.93, 69.07, 58.68;
LRMS ($ESI^+$) calculated: $C_{28}H_{26}O_{12}$, 554.14 found: $C_{28}H_{26}O_{12}$, 553.14;
elemental analysis (%) for $C_{28}H_{26}O_{12}$ calculated: C 60.65, H 4.46; found: C 52.46, H 4.73.
Compound **6**: Yield=95%;
$^1H$ NMR (500 MHz, DMSO) δ 13.31 (s, 4H), 8.45 (t, *J* = 1.7 Hz, 2H), 8.38 (d, *J* = 1.6 Hz, 4H), 7.23 (s, 2H), 4.20-4.15 (m, 4H), 3.67-3.62 (m, 4H), 3.46 (dd, *J* = 5.8, 3.8 Hz, 4H), 3.33 (d, *J* = 2.1 Hz, 4H), 3.14 (s, 6H);
$^{13}C$ NMR (126 MHz, DMSO) δ 167.13, 150.24, 138.75, 134.66, 131.62, 129.33, 129.07, 116.33, 71.60, 70.18, 69.48, 69.46, 58.37;
LRMS (ESI+) calculated: $C_{32}H_{34}O_{14}$ 642.19 found: $C_{32}H_{34}O_{14}K$ 681.16 (one K atom plus)
elemental analysis (%) for $C_{32}H_{34}O_{14}$ calculated: C 59.81, H 5.33; found: C 58.13, H 5.18.

## Synthesis and characterization of the MOFs:

The preparation of the different MOFs starting from the organic compounds according to formula (II) was performed according to the scheme reported on figure 2.

**SUM-103 and SUM-102:** In a 10 ml dram vial, 0.018 mmol ligand (compound **5** or **6**) are tared and dissolved in a mixture of 4 ml DMF and 1,3 ml water. Then, 0,072 mmol (17,5 mg) of $Cu(NO_3)_2.3H_2O$ was added to the solution and dissolved in the solution. Finally, 33 μl of concentrated HCl was added, the vial is closed and put into dry bath at 80°C. After 24h, the obtained microcrystalline powder is filtered and washed with DMF to get 14 mg (48% yield) of the MOF **SUM-103** and 12.5 mg (45% yield) of the MOF **SUM-102.**

**SUM-150** : In a 10 ml dram vial, 0.009 mmol ligand (compound **6**) are tared and dissolved in a 2 ml DMF. Then, 0.054 mmol (13.8 mg) of $Mg(NO_3)_2x6H_2O$ was added to the solution and dissolved in the solution. Finally, 2 drops 10 times diluted HCl was added, the vial is closed and put into dry bath at 120°C. After 24 to 48h, the obtained microcrystalline powder is filtered and washed with.

FT/IR spectra of MOFs with their corresponding ligands are shown in figure 3 and 4. (H4L(glyme): is for compound **5**; H4L(glyme): is for compound **6**).

Since the side chains could not be located by XRD, their presence was established by FT-IR analysis (see ESI). Indeed, C-H stretching vibrations (2890 $cm^{-1}$) and the skeletal vibrations of aromatic rings or C-C-O chains (1200 and 1505 $cm^{-1}$) demonstrated the presence of backbone and side chains.

The formation of MOFs can also be deduced from the C=O stretching band shifted to low energies due to the coordination of the carboxylate groups to Cu (1630 $cm^{-1}$). Also, Cu-O bond elongation band could be observed at 730 $cm^{-1}$.

Thermal stability (figure 5):

TGA analysis up to 450°C under nitrogen of **SUM-102** and **SUM-103** are characterized by nearly the same pattern. First phase occurs in two waves of solvents evaporation, one below 50°C for most volatile solvents and a second one between 70 and 200°C for less volatile ones. Decomposition of MOFs started at 280°C and 289°C respectively.

Therefore, addition of oxygen rich side chains does not influence the thermal stability of MOF's.

Stability in water of the two MOFs: **SUM-102** and **SUM-103** (figure 6):
The presence of ethylenoxy units confers to the **SUM-102,** and more particularly to **SUM-103** with two ethylenoxy units, an excellent resistance to water. This after treatment for 8 hours at 160°C and re-exposure to ambient air, whereas in the case of **SUM-102** a loss of crystallinity was observed, **SUM-103** appeared to be stable.

$N_2$ **adsorption** / BET surface (figure 7):

The nitrogen adsorption of **SUM-102** and **SUM-103** is displayed on figure 7. Despite larger side chains of H4L(diglyme)2 ligand, **SUM-103** demonstrates higher surface area. It can therefore be seen that the material with the longest side chains carrying two ethylenoxy units has a greater asorption capacity than the material with only one ethylenoxy unit in the side chains.

The BET surface area measured for **SUM-102** are 869 $m^2/g$ ($N_2$) versus 846 $m^2/g$ (Ar) and for **SUM-103** are 1058 $m^2/g$ ($N_2$) versus 1016 $m^2/g$ (Ar) and shows type I isotherm characteristic for microporous materials.

**$CO_2$ adsorption**

Figure 12 shows the $CO_2$ adsorption by the MOF **SUM-103** : 38,5 mmol/g adsorbed; 7 mmol/g stays (10 bars, 0°C).
Figure 13 shows the $CO_2$ adsorption by the MOF **SUM-102** : 17 mmol/g adsorbed (10 bars, 0°C).
Figure 14 shows the $CO_2$ adsorption by the MOF SUM-150 : 27 mmol/g adsorbed (10 bars, 0°C).

**Dye adsorption**

Methylene Blue adsorption :

The adsorption capacity of Methylene Blue was calculated based on Equation (1) **[4]-[6].** The equilibrium adsorption capacity of adsorbent was calculated using Equation (2) **[6].**

$$Qt = (Co - Ct).\ V/m \quad (1)$$

$$Qe = (Co - Ce).\ V/m \quad (2)$$

where Qt and Ct define the adsorption capacity of the adsorbent (mg/g) and the adsorbate concentration (mg/L), respectively. V represents the volume of adsorbate solution and m the mass of MOF adsorbent. Likewise, Qe and Ce define the adsorption capacity of adsorbent and adsorbate concentration (mg/L), respectively, at the equilibrium conditions.

The MB isotherms were fitted with Langmuir and Freundlich models in order to calculate the maximal adsorption capacity and get insights about the nature of the adsorption. Linear form of Langmuir equation is expressed as indicated below:

$$\frac{C_e}{Q_e} = \frac{1}{Q_m K_L} + \frac{C_e}{Q_m} \quad (3)$$

Ce is the equilibrium concentration, Qe is equilibrium uptake capacity. KL and Qm are obtained from the slope and the intercept of Ce/Qe vs Qe plot. R2 of the linear plot is 0.9992 which shows applicability of this model.

Besides, the separation factor-RL is calculated with Eq (4).

$$R_L = \frac{1}{1 + C_m K_L} \quad (4)$$

Cm is maximal initial concentration of methylene blue. The RL shows favorability of adsorption. The value between 0 and 1 shows good adsorption.

To fit the data to Freundlich model the Eq (5) was used:

$$\ln Q_e = \ln K_F + \frac{1}{n} \ln C_e \quad (5)$$

To find KF and 1/n (adsorption constants), the plot of lnQe vs lnCe were drawn. R2 is 0.7507 which doesn't show a good agreement of this model. The values of adsorption constants for both isotherms are summarized in Table 1.

Table 1: parameters of isotherm modelling for **SUM-103**

| Isotherm model | Constants | |
|---|---|---|
| Langmuir | $Q_m$ (mg/g) | 194 |
| | KL (L/mg) | 0,0777 |
| | $R_L$ | 0,0605 |
| | $R^2$ | 0,9992 |
| Freundlich | $K_F$ (mg/g) | 39,1629 |
| | 1/n | 0,3429 |
| | $R^2$ | 0,7507 |

In order to properly describe the adsorption process, two popular methods for studying the adsorption kinetics were applied: Pseudo-first order (PFO) and pseudo second order (PSO). Linear equation of PFO (6) and PSO (7) could be expressed as below:

$$\ln(Q_e - Q_t) = lnQ_e - k_1 t \quad (6)$$

$$\frac{t}{Q_t} = \frac{t}{Q_e} + \frac{1}{k_2 Q_e^2} \quad (7)$$

Qe and Qt are the amounts of methylene blue adsorbed (mg/g) on MOFs at equilibrium and at the time t. $k_1$($min^{-1}$) and $k_2$ (g/mg·min) are the rate constants of PFO and PSO, respectively.

| Kinetic model | Constants | |
|---|---|---|
| Pseudo First Order | $K_1$ (L/min) | 0,0042 |
| | $Q_e$ (mg/g) | 2,29 |
| | $R^2$ | 0,9959 |
| Pseudo Second Order | K2 (g/mg·min) | 0,0041 |
| | $Q_e$ (mg/g) | 15,21 |
| | $R^2$ | 0,9997 |

Figure 8: Crystals of **SUM-102** and **SUM-103** are observed under Scanning Electron Microscope. Photographs in figure 8 show for SUM-103 size around 10-20 μm, as also observed for SUM-102.

Dyes (methylene blue and alizarin yellow R) adsorption capacity of both MOFs have been evaluated. In case of **SUM-103,** both isothermal adsorption and kinetic studies were performed. The adsorption parameters have been determined in water (neutral pH) at 30°C (for isotherm). Both **SUM-102** and **SUM-103** have been compared under identical conditions. The values of dyes adsorption percentage of dye solutions (10 ml ; 10 mg/l) over 3 mg MOF after 48 h and at 30°C are given in table 2.

Table 2: adsorption of dyes versus MOFs sample (by wt% of dyes adsorbed over MOFs)

| | SUM-102 (%) | SUM-103 (%) |
|---|---|---|
| Alizarin Yellow R | 64 | 77 |
| Methylene blue | 83 | 94 |

Those values show that methylene blue is more efficiently adsorbed than alizarin yellow R. Also, we observe significant increase in adsorbance with increasing side chains length. The study of the adsorption of methylene blue (MB) on **SUM-103** was extended by measurements of isothermal adsorption capacity and a kinetic study, details of these measurements are available in SI. The adsorption protocol was carried out using the batch method by adding 20, 50, 100 and 200 ppm methylene blue solutions over 3 mg MOF in different vials (10 mL) and then placed in the oven at 30°C. After adsorption of methylene blue, the colour of the MOF's changed from blue to deep-blue. Adsorption of methylene blue on MOFs was also assessed by the presence of characteristic strong bands of methylene blue in FT-IR spectrum of final materials (figure 9: from left to the right, **SUM-103;** adsorption kinetics, 2,5 ml, 15 mg/l MB solution after 24h at room temperature, FT-IR comparison before and after adsorption). After 24h, the supernatant of methylene blue solution was analysed using a UV-VIS spectrophotometer at 664 nm. Figure 10 gives Langmuir and Freundlich modelling of MB adsorption of **SUM-103.** Figure 11 gives PFO and PSO modelling of MB adsorption of **SUM-103** at room temperature.

The isothermal adsorption capacities were modeled according to the models of Langmuir and Freundlich. It is observed that the quantity of methylene blue which binds to the material is 194 mg / g (Langmuir) for **SUM-103.**

**REFERENCES**

**[1]** Makal T. A. & al., Tuning the Moisture and Thermal Stability of Metal-Organic Frameworks through Incorporation of Pendant Hydrophobic Groups Crystal growth & Design 2013 13(11), 4760-4768.

**[2]** Pasad T. K. & al., Metal-Organic Frameworks Incorporating Various Alkoxy Pendant Groups: Hollow Tubular Morphologies, X-ray Single-Crystal Structures, and Selective Carbon Dioxide Adsorption Properties Chem. Asian J. 2015, 10, 2257-2263.

**[3]** Vazquez-Molina D. A. & al., Chemical Communications, 2018, 54, 6947-6950.

**[4]** Feng Y. & al., RSC Adv., 2016, 6, 109608-109612.

**[5]** Santoso E. & al., Microporous and Mesoporous Materials, 2021, 310, 110620.

**[6]** Peres E. C. & al., J. Environ. Chem. Eng., 2018, 6, 649-659.


**Claims**

1. Organic compound for the preparation of a MOF, said organic compound being selected among the compounds according to formula (I):

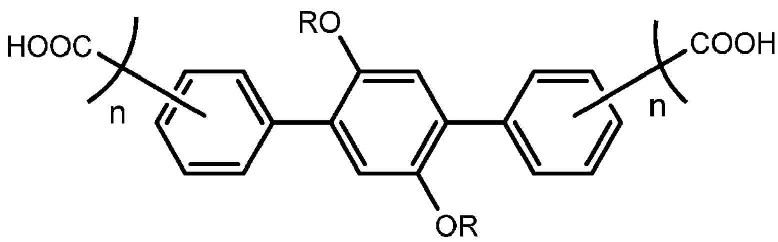

(I)

- wherein n is from 1 to 2,
- R is $-(CH_2-CH_2-O)_m-CH_3$ with m from 1 to 7;

preferably when n=1, the two -COOH groups are located in the para position relatively to the aryl substituted by the two RO groups; and
preferably when n=2, the four -COOH groups are located in the meta position relatively to the aryl substituted by the two RO groups.

2. Organic compound according to claim 1, wherein n is 2 ; the organic compound being selected among the compounds according to formula (II):

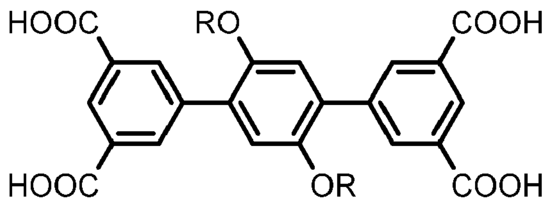

(II)

**3.** Organic compound according to claim 2, wherein m is less than 4.

**4.** Metal-organic framework material (MOF), comprising at least one metal center and at least one organic compound according to any of claim 1 to 3.

**5.** Metal-organic framework material according to claim 4, wherein the metal center of the hydrophobic core is selected from Li, Na, Rb, Mg, Ca, Sr, Ba, Sc, Ti, Zr, Ta, Cr, Mo, W, Mn, Fe, Ru, Os, Co, Ni, Pd, Pt, Cu, Au, Zn, Al, Ga, In, Si, Ge, Sn, Bi, Cd, Mn, Tb, Gd, Ce, La, or Cr, and preferably Cu, Zn, Zr, Ca or Mg.

**6.** Metal-organic framework material according to claim 5, wherein the organic compound is :

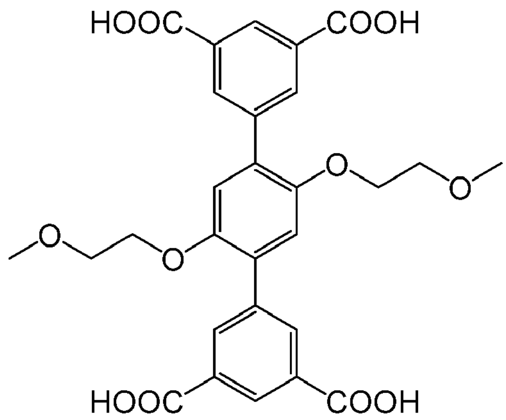

and the metal center is selected from Cu, Zn, Mg, Ni and Ca.

**7.** Metal-organic framework material according to claim 5, wherein the organic compound is selected among :

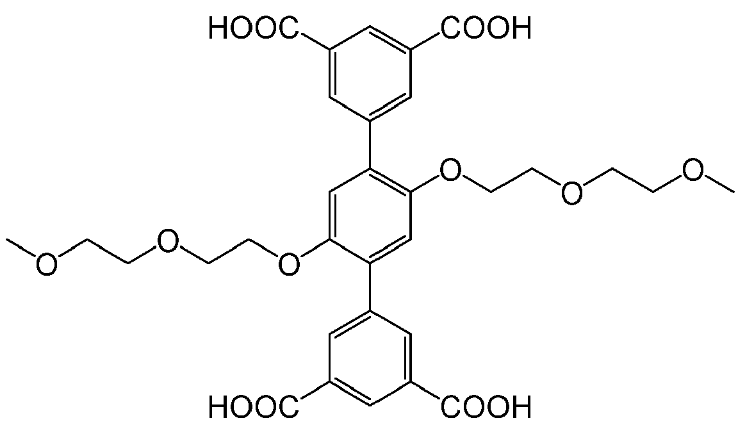

,

and

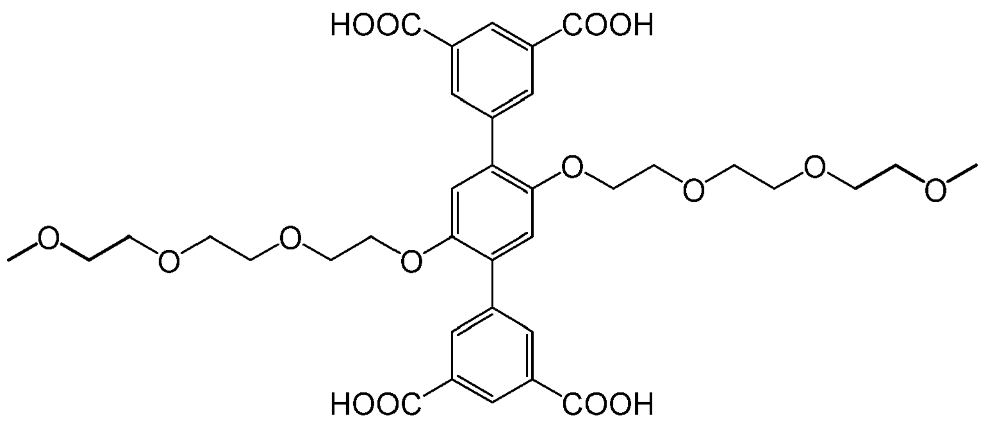

and the metal center is selected from Cu, Zn, Mg, Ni and Ca.

8. Metal-organic framework material according to claim 4 or 5, wherein the organic compound is :

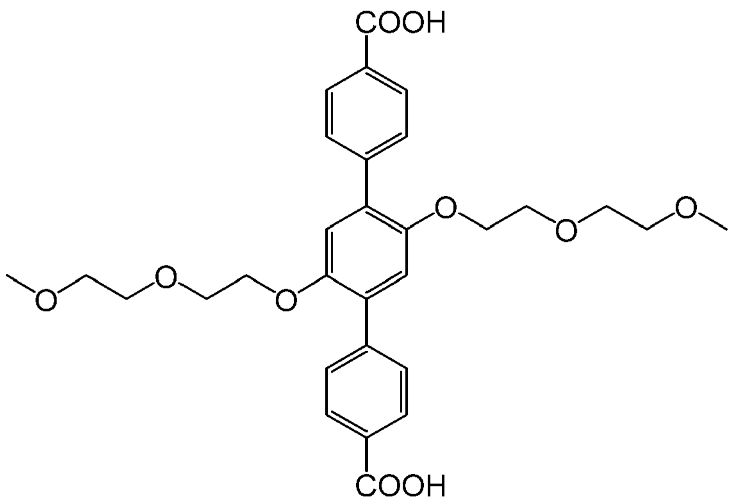

and the metal center is selected from Cu, Zr, Zn, Fe and Ca.

9. Use of a MOF according to any of claims 4 to 8, for the adsorption of volatile organic compounds (VOCs), and preferably for the adsorption of acetic acid or aldehydes.

10. Use of a MOF according to any of claims 4 to 8, for the decontamination of aqueous solutions containing organic pollutants such as dyes and pharmaceutical compounds, and preferably for the adsorption of a dye, preferably selected among a cationic and an anionic dye, and most preferably selected among at least methylene blue and alizarin yellow R.

11. Use of a MOF according to any of claims 4 to 8, for the adsorption of a gas, preferably selected among methane, hydrogen, acetylene, nitrogen, and $CO_2$, most preferably $CO_2$.

12. Use of a MOF according to claim 11, for the adsorption of $CO_2$ wherein the concentration of adsorbed gas is greater than 17 mmol/g at 10 bars, and 0°C.

13. Preparation method for the synthesis of a MOF according to any of claims 4 to 8, wherein the metal center is Cu, and the MOF is prepared by the dissolution of the organic compound according to any of claims 1 to 3, in a DMF/water mixture, followed by an addition plus dissolution of $Cu(NO_3)_2.3H_2O$ into the reaction mixture, the reaction mixture is left at 70-90°C during several hours.

14. Preparation method for the synthesis of the organic compound according to any of claims 1 to 3, comprising the

steps of :

a- activating phenol groups of a hydroquinone bearing two halogen or two -OTf groups ;
b- performing a Suzuki-Miyaura reaction to replace halogen and/or -OTf by aryl groups bearing protected acid acetic substituents ; and
c- deprotecting the acid acetic substituents;

preferably according to the following synthetic pathway:

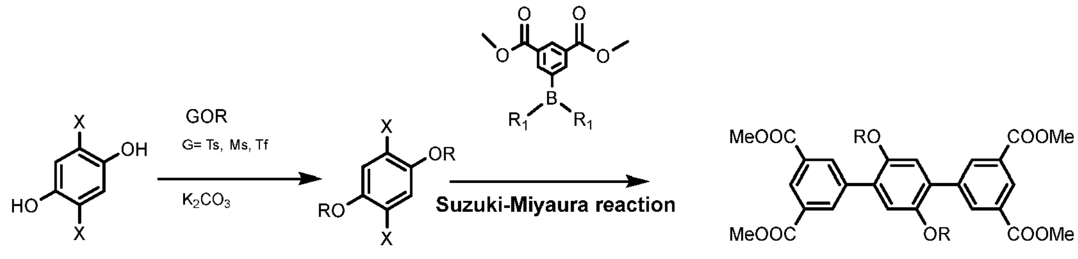


X=halogen, OTf

$R_1$=OH, $OR_2$ ($R_2$=alkyl), alkyl , and/or
$2R_1$-B or $2(OR_2)$-B forming together a cycle

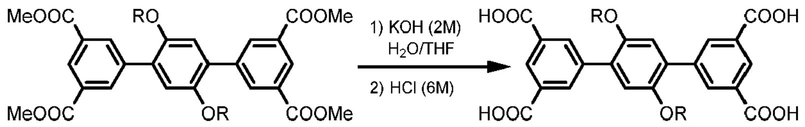


**(II)**

the R groups been as defined in claim 1.

**15.** Preparation method according to claim 14, wherein X=Br; G=Ts and the first step of the synthetic pathway is realized in DMF at 80°C, and the Suzuki-Miyaura coupling reaction step is performed according to:

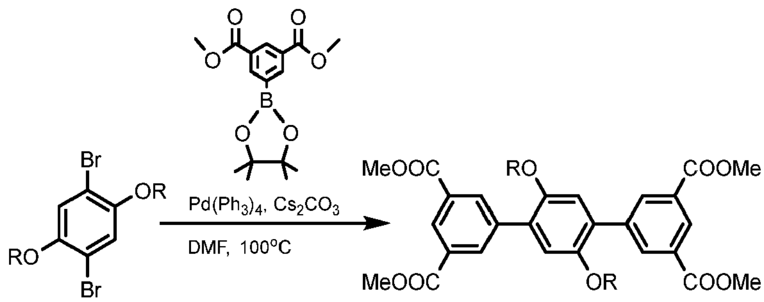


**(II)**

FIG. 1

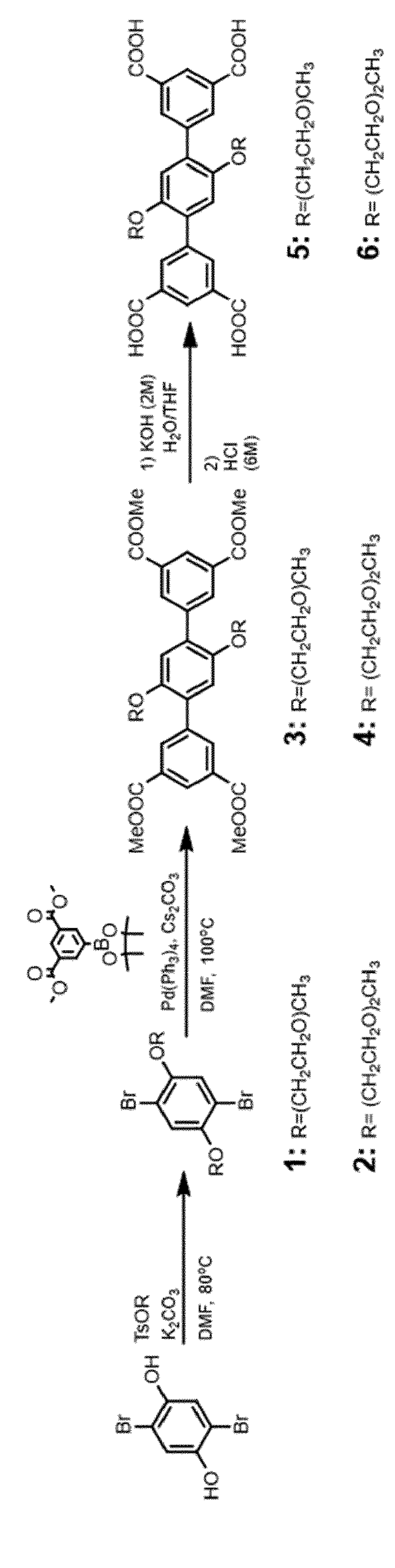


**1:** R=$(CH_2CH_2O)CH_3$

**2:** R= $(CH_2CH_2O)_2CH_3$

**3:** R=$(CH_2CH_2O)CH_3$

**4:** R= $(CH_2CH_2O)_2CH_3$

**5:** R=$(CH_2CH_2O)CH_3$

**6:** R= $(CH_2CH_2O)_2CH_3$

FIG. 2

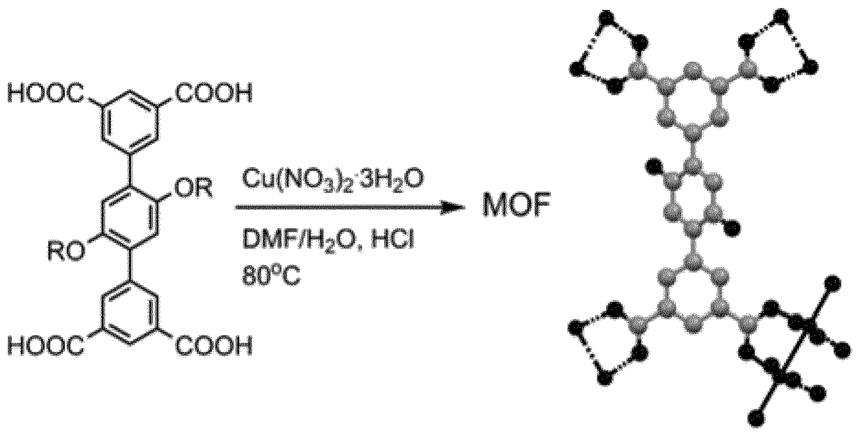


**SUM-102**
**SUM-103**

**SUM-102**, R= $-(CH_2-CH_2-O)-CH_3$; **SUM-103**, R= $-(CH_2-CH_2-O)_2-CH_3$

FIG. 3

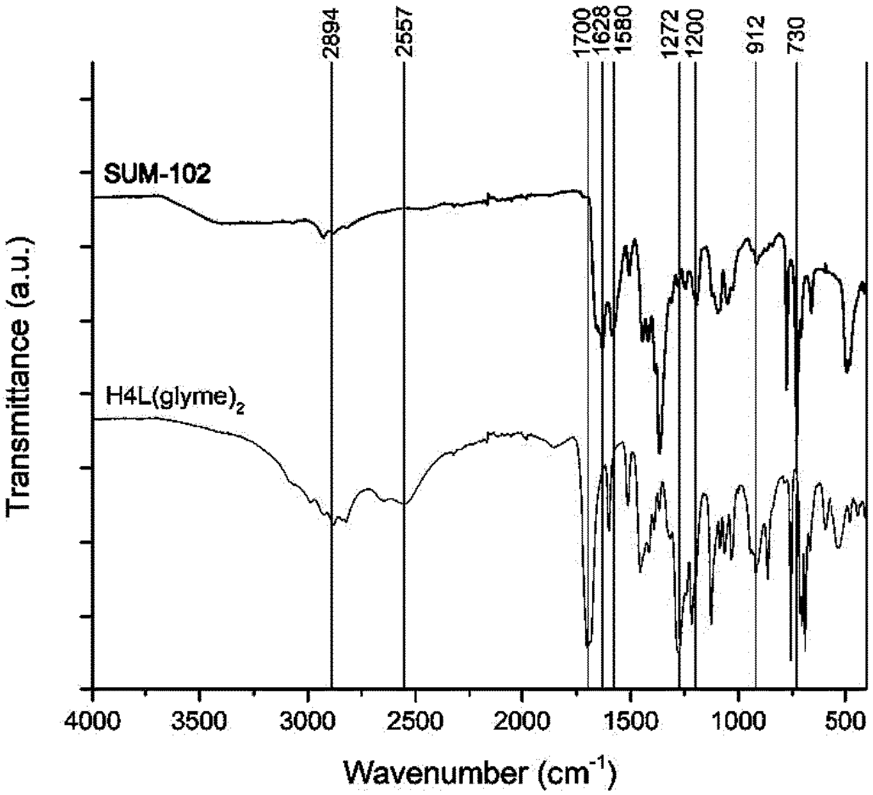
2894
2557
1700
1628
1580
1272
1200
912
730
SUM-102
H4L(glyme)2
Transmittance (a.u.)
4000
3500
3000
2500
2000
1500
1000
500
Wavenumber (cm-1)

FIG. 4

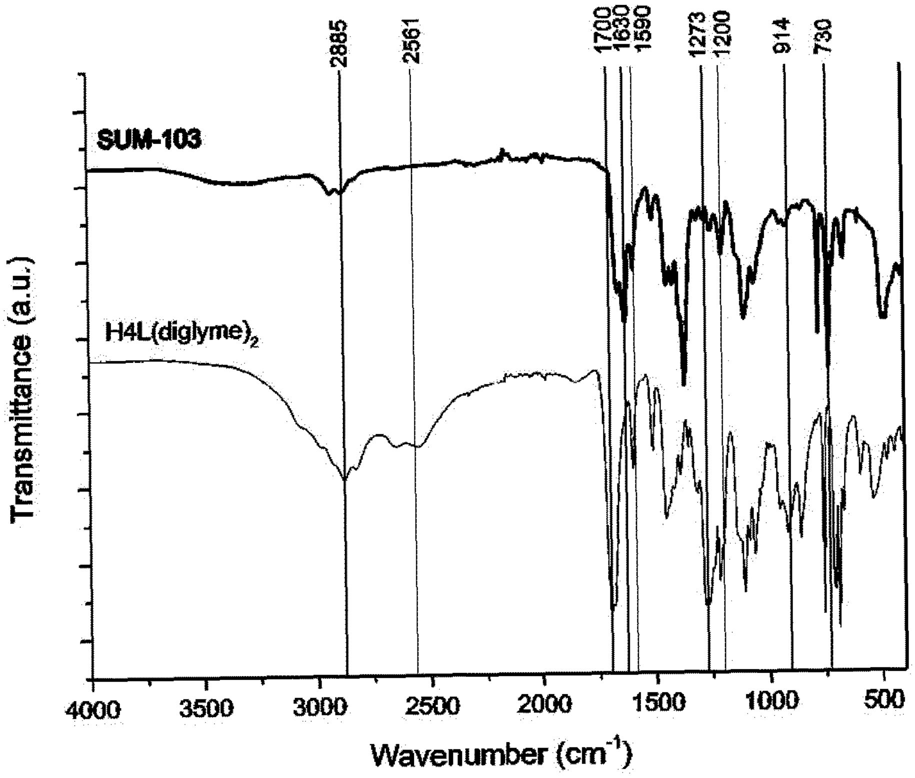
2885
2561
1700
1630
1590
1273
1200
914
730
SUM-103
H4L(diglyme)$_2$
Transmittance (a.u.)
4000
3500
3000
2500
2000
1500
1000
500
Wavenumber (cm$^{-1}$)

FIG. 5

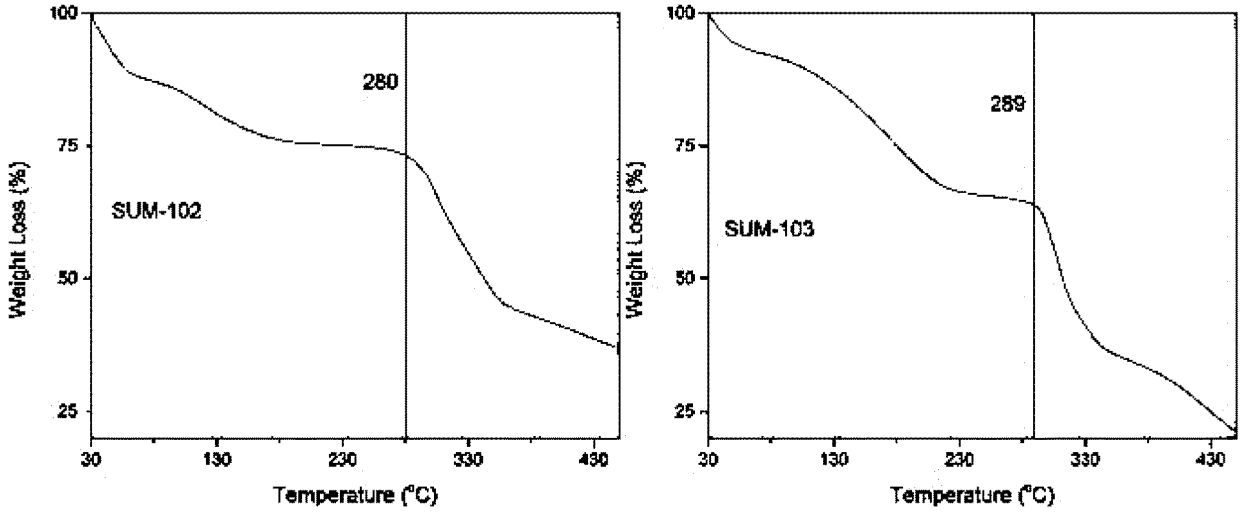
100
75
50
25
Weight Loss (%)
280
SUM-102
30
130
230
330
430
Temperature (°C)
100
75
50
25
Weight Loss (%)
289
SUM-103
30
130
230
330
430
Temperature (°C)

FIG. 6

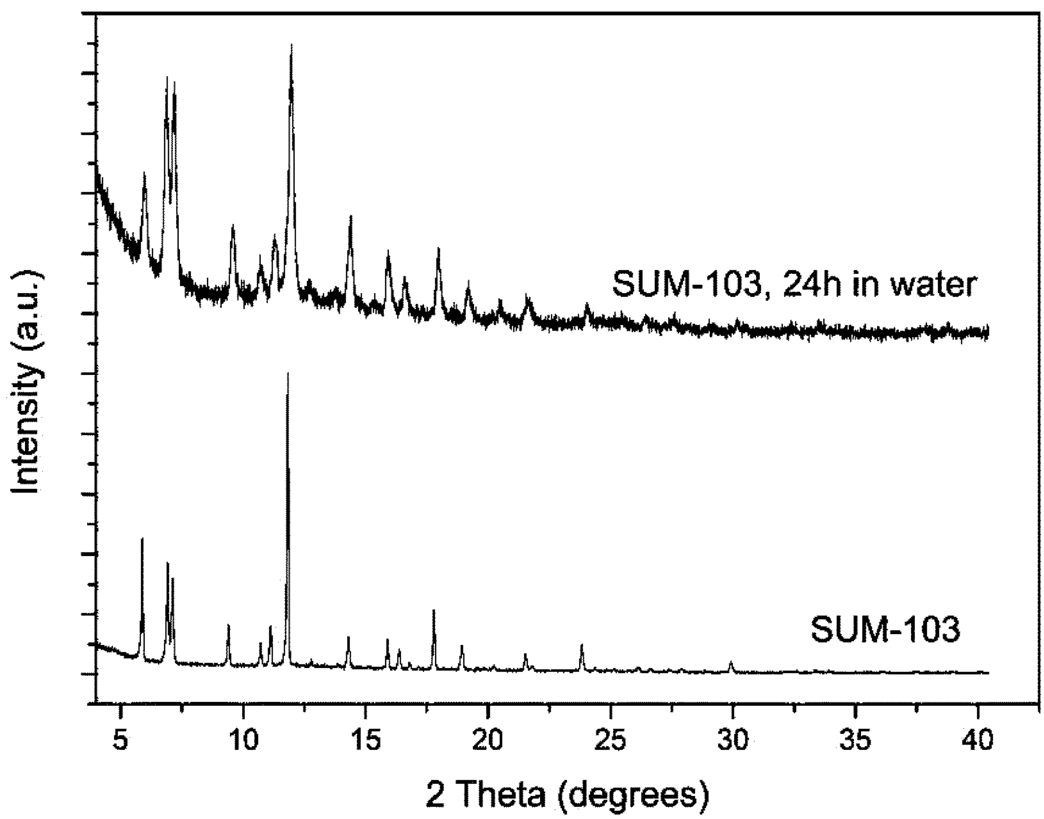
Intensity (a.u.)
SUM-103, 24h in water
SUM-103
5
10
15
20
25
30
35
40
2 Theta (degrees)

FIG. 7

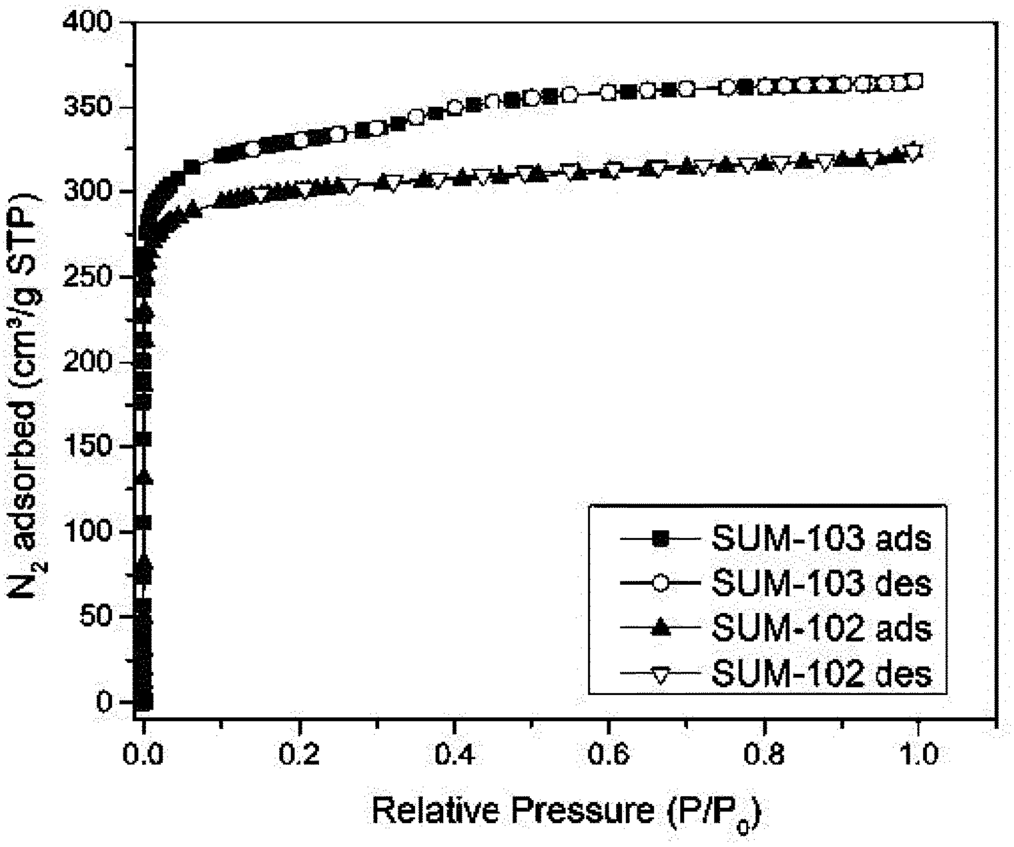
N2 adsorbed (cm3/g STP)
Relative Pressure (P/P0)
0
50
100
150
200
250
300
350
400
0.0
0.2
0.4
0.6
0.8
1.0
SUM-103 ads
SUM-103 des
SUM-102 ads
SUM-102 des

FIG. 8

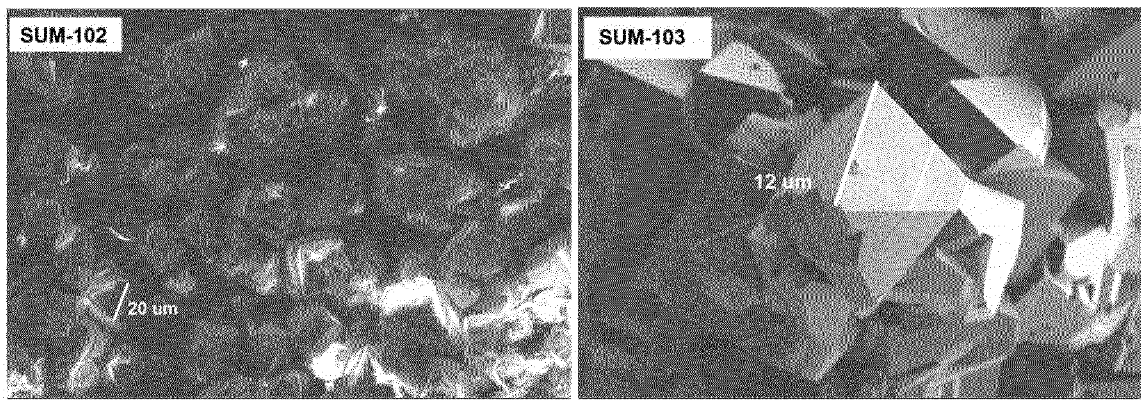
SUM-102
20 um
SUM-103
12 um

FIG. 9

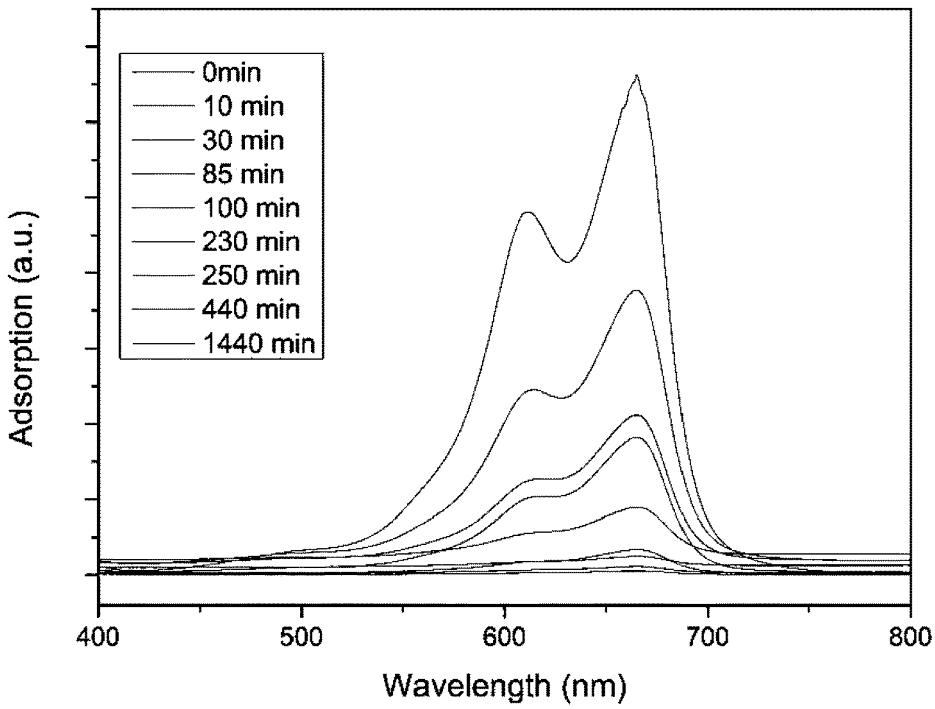
0min
10 min
30 min
85 min
100 min
230 min
250 min
440 min
1440 min
Adsorption (a.u.)
400
500
600
700
800
Wavelength (nm)

FIG. 10

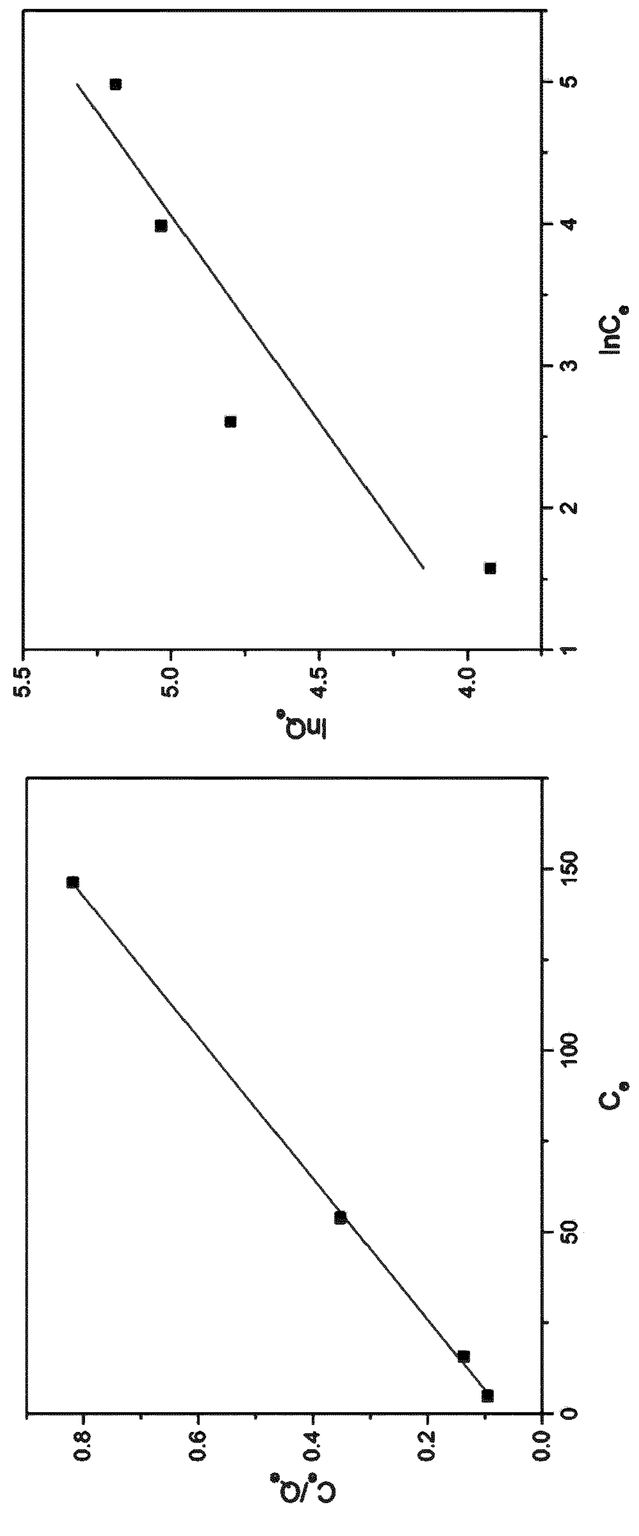

FIG. 11

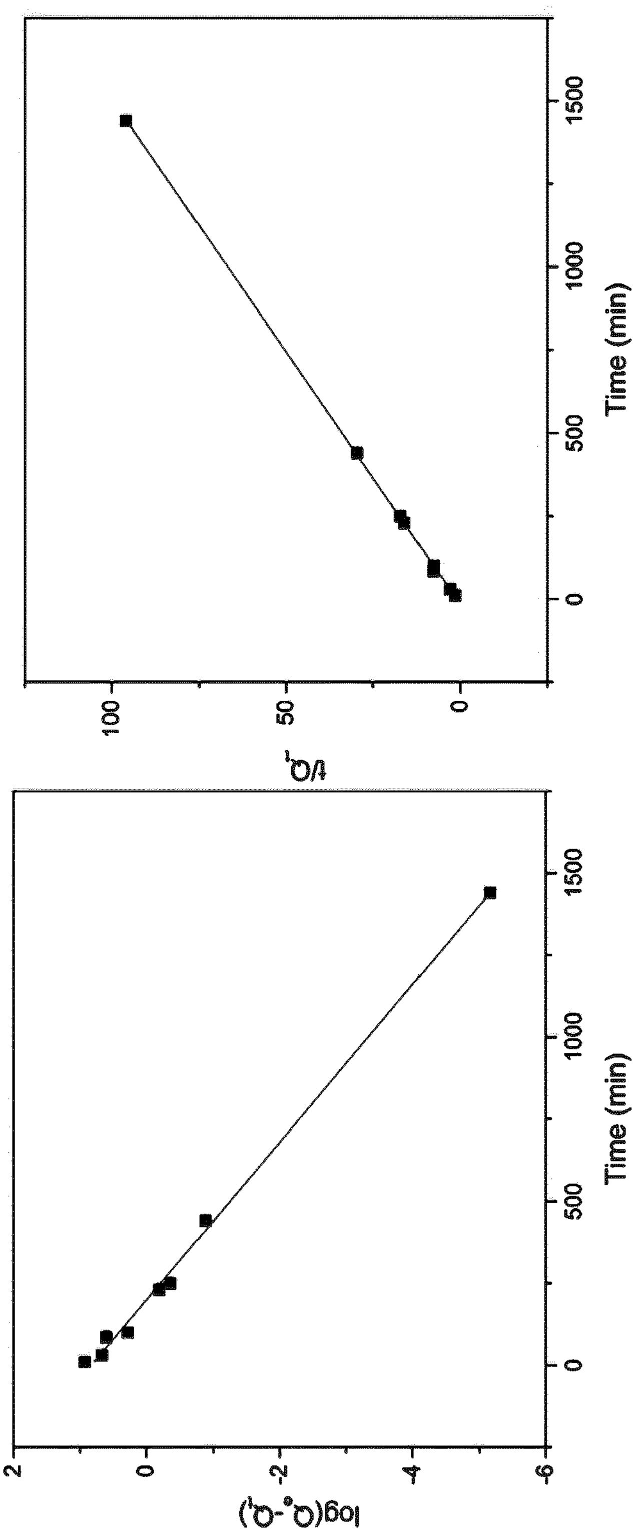
t/Q_t
100
50
0
Time (min)
0
500
1000
1500
log(Q_e-Q_t)
2
0
-2
-4
-6
Time (min)
0
500
1000
1500

FIG. 12

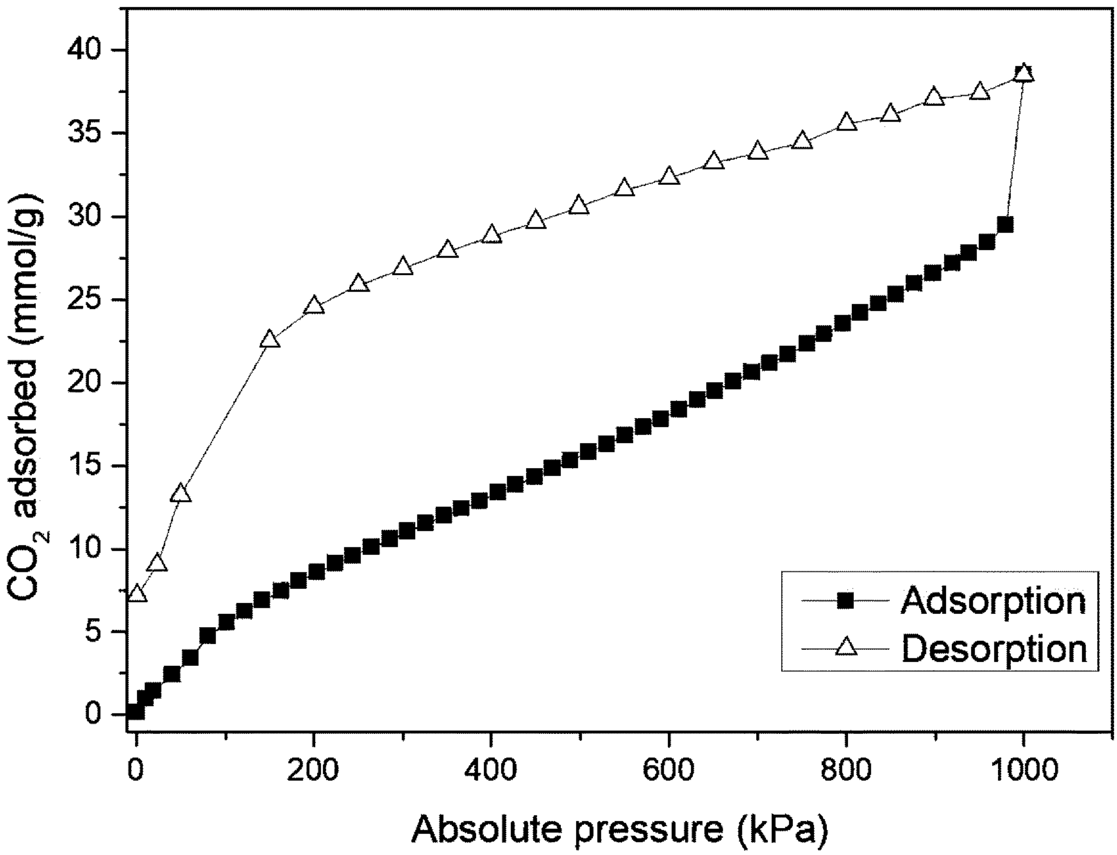
40
35
30
25
20
15
10
5
0
$CO_2$ adsorbed (mmol/g)
0
200
400
600
800
1000
Absolute pressure (kPa)
Adsorption
Desorption

FIG. 13

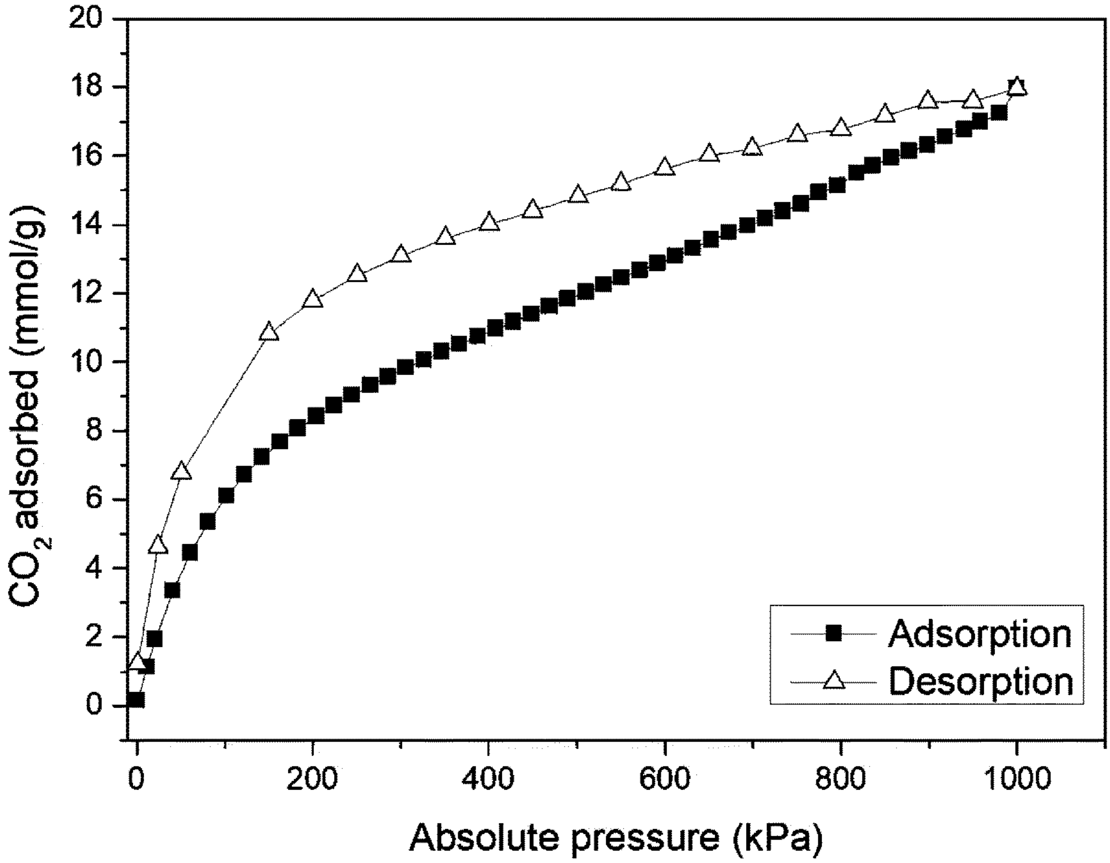
20
18
16
14
12
10
8
6
4
2
0
$CO_2$ adsorbed (mmol/g)
0
200
400
600
800
1000
Absolute pressure (kPa)
Adsorption
Desorption

FIG. 14

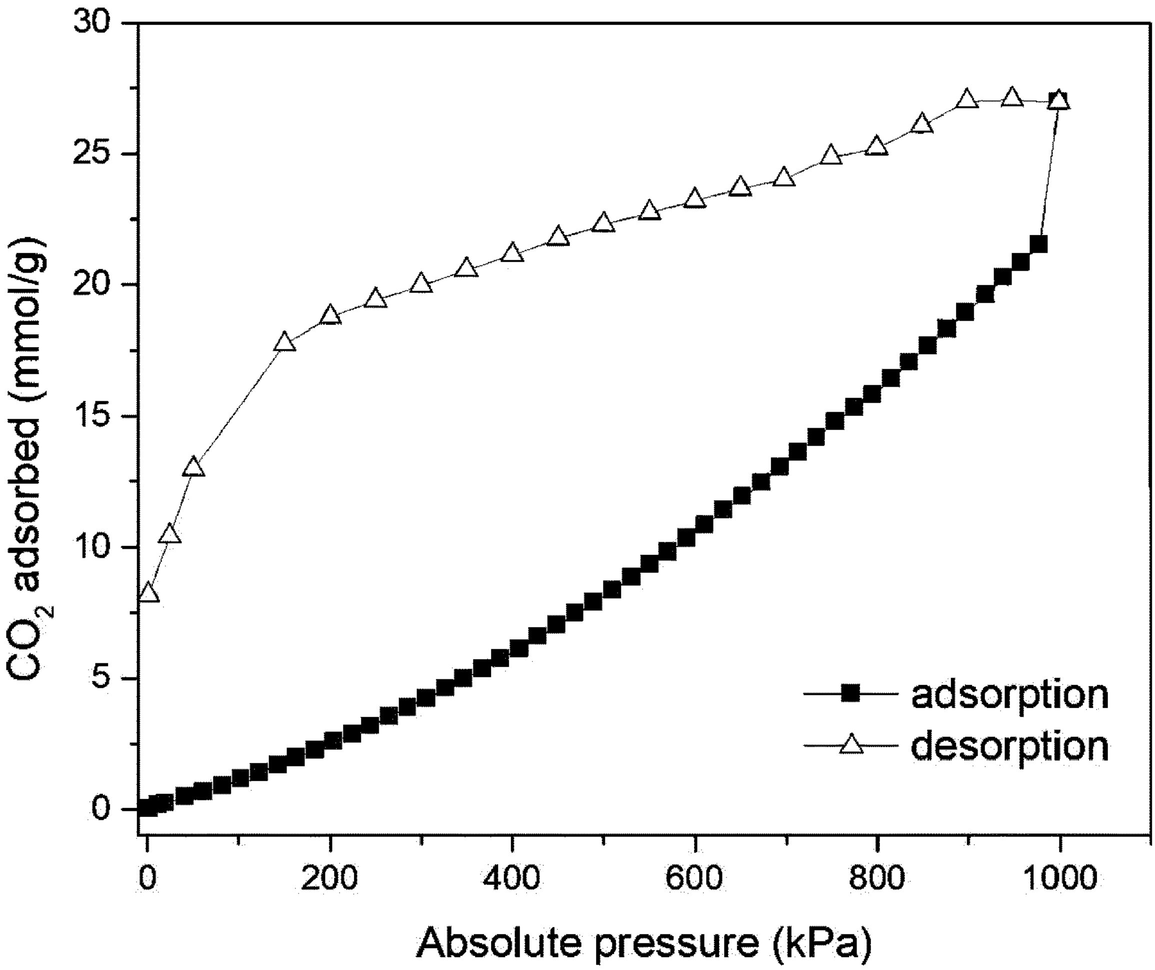
30
25
20
15
10
5
0
$CO_2$ adsorbed (mmol/g)
0
200
400
600
800
1000
Absolute pressure (kPa)
adsorption
desorption

Europäisches Patentamt
European Patent Office
Office européen des brevets

# EUROPEAN SEARCH REPORT

Application Number
EP 21 30 6902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/122297 A1 (GRUNDER SERGIO [US] ET AL) 16 May 2013 (2013-05-16)<br>* last compound page 4 *<br>----- | 1-15 | INV.<br>C07F1/00<br>C07F3/00<br>C07C63/331<br>C07C65/105<br>C07C65/24<br>B82Y40/00 |

TECHNICAL FIELDS SEARCHED (IPC)

C07F
B82Y
C07C

The present search report has been drawn up for all claims

1

| Place of search | Date of completion of the search | Examiner |
|---|---|---|
| The Hague | 7 June 2022 | Bourghida, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 21 30 6902

This annex lists the patent family members relating to the patent documents cited in the above-mentioned European search report. The members are as contained in the European Patent Office EDP file on The European Patent Office is in no way liable for these particulars which are merely given for the purpose of information.

07-06-2022

| Patent document cited in search report | Publication date | Patent family member(s) | Publication date |
|---|---|---|---|
| US 2013122297 A1 | 16-05-2013 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MAKAL T. A.** Tuning the Moisture and Thermal Stability of Metal-Organic Frameworks through Incorporation of Pendant Hydrophobic Groups. *Crystal growth & Design,* 2013, vol. 13 (11), 4760-4768
- **PASAD T. K.** Metal-Organic Frameworks Incorporating Various Alkoxy Pendant Groups: Hollow Tubular Morphologies, X-ray Single-Crystal Structures, and Selective Carbon Dioxide Adsorption Properties. *Chem. Asian J.,* 2015, vol. 10, 2257-2263
- **VAZQUEZ-MOLINA D. A.** *Chemical Communications,* 2018, vol. 54, 6947-6950
- **FENG Y.** *RSC Adv.,* 2016, vol. 6, 109608-109612
- **SANTOSO E.** *Microporous and Mesoporous Materials,* 2021, vol. 310, 110620
- **PERES E. C.** *J. Environ. Chem. Eng.,* 2018, vol. 6, 649-659